# EUROPEAN PATENT APPLICATION

(11) **EP 0 901 795 A2**
(43) Date of publication of application: **17.03.1999**
(21) Application number: 98307327.1
(22) Date of filing: 10.09.1998
(51) Int. Cl.: A61L 15/32, A61L 15/20, A61L 27/00

(54) **Buffered wound dressing materials**

(30) Priority: 11.09.1997 GB 9719358
(71) Applicant: JOHNSON & JOHNSON MEDICAL, INC., Arlington, Texas 76004-3030 (US)
(72) Inventor: Watt, Paul William, Steeton, West Yorkshire BD20 6UN (GB); Tarlton, John, Horfield, Bristol BS7 8QY (GB)
(74) Representative: James, Anthony Christopher W.P.

(57) **Abstract**

The invention provides solid bioabsorbable materials comprising a weak, watersoluble acid buffer system dissolved or dispersed in a protein matrix in an amount of at least 0.1 mmol/g based on the weight of the material. The buffer system comprises a weak, water-soluble, physiologically acceptable acid and a water-soluble, physiologically acceptable conjugate base of a weak acid. The material is preferably used as or in wound dressings in order to maintain wound surfaces at acid pH, preferably pH 3.5 to 6.5 Preferably, the material is a collagen sponge having a solid hydrogen phosphate/dihydrogen phosphate buffer dispersed therein.

## Description

The present invention relates to solid bioabsorbable materials, wound dressings and implants comprising such materials, and the use of such materials in the preparation of wound dressings and implants.

It is known to use bioabsorbable polymeric materials for the preparation of wound dressings, soft tissue implants and temporary prostheses. Suitable materials include synthetic bioabsorbable polymers such as the polymers and copolymers of lactic acid, glycolic acid, p-dioxanone and ε-caprolactone. Other suitable bioabsorbable polymers include biopolymers and modified biopolymers, such as collagen and oxidized regenerated cellulose. These materials have a high level of biological acceptability, and break down completely *in vivo* into low molecular weight, biologically absorbable components. Such bioabsorbable materials are particularly suitable for use as or in wound dressings, because they avoid the trauma caused by the removal of conventional wound dressing materials such as non-resorbable fabrics from the surface of the healing wound. In addition, certain materials such as collagen are thought to have a positive therapeutic effect on wound healing.

A drawback of some collagen-based wound dressing materials is that the collagen breaks down too fast *in vivo,* due to the action of collagenase enzymes in the wound. This can be countered to some extent by cross-linking the collagen with a covalent cross-linking agent such as glutaraldehyde or dicyclohexylcarbodiimide. Nevertheless, a need remains for improved collagen-based wound dressings.

A number of studies have been carried out on the pH of the wound environment during wound healing. Whilst it is no simple matter to determine the actual pH at a wound site, it appears that the pH of chronic wounds is slightly neutral or slightly alkaline, whereas the pH of intact skin is slightly acidic (pH 4 or 5).

I.A.I. Wilson *et al.,* in VASA Band 1979 Heft 4, pages 339-342 describe an investigation of the pH of varicose ulcer surfaces, and the relationship of pH to healing. The wound surface pH of fifty varicose ulcers was measured using a Cecar (RTM) micro-combination pH electrode. All fifty ulcers measured were in the alkaline region, in the range pH 7.3-8.9, with a mean of 7.7. It was found that prolonged acidification of the wound surfaces using acid buffered solutions increased the healing rate. However, it is not practical or desirable to irrigate chronic wounds with acid-buffered solutions in normal medical practice.

GB-A-2082911 describes pharmaceutical ointments for application to wounds to assist wound healing. The ointments contain a mixture of amino acids and malic acid and have a pH in the range 6.5 to 8.

EP-A-0564307 describes diapers, sanitary napkins, pads, dry compositions and creams containing buffering compositions for the reduction of skin irritations due to contact with excreted body fluids such as urine. The compositions contain acid buffers to maintain the pH of the excreted body fluids within the range of from about 4.5 to about 6.0. In this pH range, the activity of urease enzymes is suppressed, which reduces the tendency of excreted body fluids to cause rashes on exposed skin. There is no disclosure of bioabsorbable materials containing the acid buffers, and no disclosure of wound dressings containing the acid buffers.

US-A-4813942 describes a debridement wound dressing comprising a hydrocolloid wound contacting layer containing 35% to 50% w/w of pectin or other hydrocolloids capable of reducing the pH of the wound/dressing interface to 4.8-6.5. The debridement dressing is left in place for 24-48 hours, and is then replaced by a regeneration wound dressing that provides near-neutral pH at the wound surface.

It is known to provide wound dressings and implants based on acid-swollen collagen. These materials are prepared by dispersing or dissolving collagen fibers in an aqueous solution of a biologically acceptable acid such as citric acid at a pH typically of 3.5 to 5.5 in order to swell the collagen fibers, followed by removal of water by evaporation, solvent-drying or freeze-drying to give a solid acid-swollen collagen material. These materials are weakly acidic, but have only limited buffering capacity which prevents them from providing a constant, optimised pH for extended periods under physiological conditions.

It is an object of the present invention to provide improved bioabsorbable materials for use as or in wound dressings and implants.

It is a further object of the present invention to provide bioabsorbable materials that can maintain a slightly acidic pH at the surface of a wound to assist wound healing.

It is a further object of the present invention to provide solid protein-based wound dressings that are resorbed relatively slowly *in vivo.*

Accordingly, the present invention provides a solid bioabsorbable material, wherein the material comprises a weak, water-soluble acid buffer system dissolved or dispersed in a protein matrix in an amount of at least 0.1 mmol/g based on the weight of the material.

Preferably, the acid buffer system is present in an amount of at least 0.2 mmol/g, more preferably at least 0.3 mmol/g. The upper limit of the amount of acid buffer system is preferably about 5.0 mmol/g, more preferably about 2.0 mmol/g. The amount of acid buffer system is calculated as the total weight of water-soluble weak acid plus the total weight of water-soluble conjugate base. The amount is based on the weight of the solid bioabsorbable material.

The acid buffer system is a combination of one or more physiologically acceptable weak water-soluble acids with one or more physiologically acceptable water-soluble conjugate bases of weak acids. Preferably, the conjugate base is a conjugate base of the weak acid present in the system. Preferably, the acid components of the water-soluble acid buffer system have a pKₐ in the range of from 3.5 to 6.5.

The presence of a buffer system comprising both a weak acid and a conjugate base enables the materials according to the present invention to maintain a stable pH in a wound environment over an extended period of time during which gradual neutralisation of the acid is taking place, in accordance with well known physicochemical principles. For good buffering performance, the molar ratio of acid to base in the buffer system should be in the range of from 1:10 to 10:1, preferably from 1:5 to 5:1, and more preferably from 2:5 to 5:2.

Preferably, the water-soluble acid has a low vapour pressure, and more preferably it is solid when anhydrous at room temperature. Preferably, the water-soluble acid has a solubility of at least 10 mg/ml at 25°C in water, more preferably at least 20 mg/ml, and still more preferably at least 50 mg/ml.

Wound dressings based on macromolecular acids such as alginic acid, certain viscose fibres, or oxidized regenerated cellulose are known. However, these macromolecular acids have limited solubility, limited buffering capacity and provide little ability to control the pH at a wound surface. The water-soluble acid according to the present invention is preferably not a macromolecular substance.

The characterising feature of the solid bioabsorbable materials according to the present invention is the presence of a weak, water-soluble acid buffer system therein to provide a mildly acidic (preferably pH 3.5 to 5.5) environment at the wound surface, continuously as the bioabsorbable wound dressing material breaks down. Accordingly, the water-soluble acid component of the buffer system preferably has a pKₐ in the range of from 3 to 8, more preferably from 3.5 to 6.5. The solid bioabsorbable materials according to the present invention achieve pH buffering at the wound surface. This is achieved by providing a conjugate base of the water-soluble acid, or of a different physiologically acceptable, weak water-soluble acid. Preferably, the conjugate base is the conjugate base of the water-soluble acid present in the material, for example a salt of the weak water-soluble acid, more preferably a sodium salt thereof.

Preferably, the weak water-soluble acid and conjugate base are selected from the group consisting of water-soluble organic carboxylic acids and dihydrogen phosphate salts, and mixtures and conjugate bases thereof. The preferred organic carboxylic acids include C₂-C₆ alkanoic acids, benzoic acid, substituted benzoic acids, citric acid, tartaric acid, malic acid, lactic acid, succinic acid, and ascorbic acid. It goes without saying that the water-soluble acid and conjugate base used in the materials according to the present invention should be fully biologically and pharmaceutically acceptable for topical application to a wound.

The concentration of the weak, water-soluble acid in the buffer component of the bioabsorbable material according to the present invention will depend on the particular application, the nature of the wound to which the material is to be applied, and the rate of breakdown of the protein matrix in use.

The acid buffering capacity of the material according to the present invention can be determined, for example, by slurrying 1 g of the material in 10 mls of deionised water and measuring the amount of alkali (sodium hydroxide) needed to raise the pH of the slurry to 10. The acid buffering capacity of the material is preferably at least 0.05 mmol/g, more preferably at least 0.1 mmol/g, and most preferably at least 0.2 mmol/g.

The bioabsorbable material according to the present invention preferably provides a pH at the wound site in use in the range of from 3.5 to 6.5. The ability of the material to do this can be assessed, for example, by immersing the material in a small quantity of physiological saline at 25°C for a period sufficient to establish equilibrium, followed by measuring the pH of the saline. Preferably, this pH is in the range of from 3.5 to 6.5, more preferably from 4.5 to 6.5.

Preferably, the water-soluble acid and the conjugate base are both non-volatile. More preferably, both materials are solids when pure and anhydrous at 25°C. The most preferred buffer system is hydrogen phosphate/dihydrogen phosphate. Other highly preferred systems are citric acid/citrate and lactic acid/lactate. Sodium salts or sodium/potassium mixed salts of the conjugate bases are preferred.

Preferably, the protein matrix comprises a structural protein selected from the group consisting of all collagen types, fibrin, elastin, laminin and fibronectin. More preferably, the protein matrix comprises insoluble, fibrous collagen, and most preferably the protein matrix consists essentially of insoluble, fibrous collagen. The protein may be complexed with a polyanionic polysaccharide such as oxidized regenerated cellulose (ORC), for example the collagen-ORC complex that is available under the Registered Trade Mark FIBRACOL (Johnson & Johnson Medical, Inc.). In other preferred embodiments the protein may be cross-linked, for example by glutaraldehyde or dicyclohexylcarbodiimide.

Preferably, the solid bioabsorbable material comprises at least 50% w/w of protein, more preferably at least 75% w/w, and most preferably at least 90% w/w of protein.

The solid bioabsorbable protein matrix of the materials according to the present invention may be in the form of a film or a web. Preferably, the protein matrix is in the form of a freeze-dried or solvent-dried sponge, for example a collagen sponge manufactured as described in WO85/04413 or US-A-3157524 (the entire contents of which are incorporated herein by reference).

The present invention also provides a wound dressing or implant comprising a solid bioabsorbable material according to the invention. The present invention also provides the use of such materials for preparation of a dressing or implant for the treatment of a wound, preferably a chronic wound such as a venous ulcer, a pressure sore or a decubitus ulcer.

The present invention also provides a method for the treatment of a chronic wound such as a venous ulcer, a pressure sore, a diabetic ulcer or a decubitis ulcer by applying thereto a wound dressing comprising a bioabsorbable material in accordance with the present invention.

Without wishing to be bound by any theory, it is thought that the buffered wound dressing materials according to the present invention achieve enhanced wound healing, especially of chronic wounds, by lowering the pH of the wound to a range in which matrix metalloproteinase (MMP) enzymes are substantially inactive. Since the healing of chronic wounds depends critically on the balance between tissue regeneration and tissue breakdown by proteinase enzymes, a reduction in the activity of the MMP enzymes can result in a significant enhancement of wound healing. At the same time, maintaining the pH of the wound surface above about 3.5-4.5 avoids stinging sensation, and also avoids inhibiting the activity of the growth factors responsible for tissue regeneration.

A further advantage of the wound dressing materials according to the present invention is that resorption of the protein matrix *in vivo* is slower than for the corresponding matrix not having the acid buffer dispersed therein. This is because the acidic pH reduces the activity of the proteinase enzymes that break down the protein matrix *in vivo.* Thus, the present invention provides an additional, or alternative, means to cross-linking for the reduction of resorption rates *in vivo.*

Specific embodiments of the present invention will now be described further, by way of example.

### Example 1

The preparation of a buffered collagen sponge for use as a wound dressing is carried out as follows.

Milled collagen fibres prepared in accordance with US patents US-A-4614794 or US-A-4320201 (the entire contents of which are incorporated herein by reference) are added to a solution of 5 mM NaH₂PO₄/Na₂HPO₄ buffer at pH 5.0 and a concentration of 1% collagen w/v. The resulting suspension is homogenised in a blender for 3 x 30 seconds. The slurry is degassed under vacuum to remove air bubbles and poured into a mould 4 mm deep. The slurry is blast frozen at -30°C, and freeze-dried at -30°C to 20°C overnight. The resulting material is a buffered, white collagen sponge.

In variations on this method, the buffered collagen slurry may be dried by solvent drying as described in US-A-3157524 (the entire contents of which are incorporated herein by reference). The collagen slurry may also be cross-linked by a conventional cross-linking agent such as hexamethylene diisocyanate (HMDI).

The above specific embodiment has been described by way of example only. Many other embodiments falling within the scope of the accompanying claims will be apparent to the skilled reader.

## Claims

1. A solid bioabsorbable material, wherein the material comprises a weak water-soluble acid buffer system dissolved or dispersed in a protein matrix in an amount of at least 0.1 mmol/g, based on the weight of the material.

2. A solid bioabsorbable material according to claim 1, wherein the weak water-soluble acid buffer system is present in an amount of at least 0.2 mmol/g.

3. A solid bioabsorbable material according to claim 1 or 2, wherein the weak water-soluble acid buffer system is present in an amount of from 0.3 to 2.0 mmol/g.

4. A solid bioabsorbable material according to claim 3, wherein the acid component(s) of the water-soluble acid buffer has/have a pKₐ in the range of from 3.5 to 6.5.

5. A solid bioabsorbable material according to any preceding claim, wherein the material has an acid buffering capacity of at least 0.05 mmol/g.

6. A solid bioabsorbable material according to claim 5, wherein the material has an acid buffering capacity of at least 0.1 mmol/g.

7. A solid bioabsorbable material according to any preceding claim, wherein the weak water-soluble system is selected from the group consisting of water-soluble organic carboxylic acids and dihydrogen phosphate salts, and mixtures thereof in combination with one or more conjugate bases thereof.

8. A solid bioabsorbable material according to claim 7, wherein said organic carboxylic acids are selected from the group consisting of C₂-C₆ alkanoic acids, benzoic acid, substituted benzoic acids, citric acid, tartaric acid, malic acid, lactic acid, succinic acid and ascorbic acid.

9. A solid bioabsorbable material according to any preceding claim, wherein said weak water-soluble buffer system comprises sodium hydrogen phosphate/sodium dihydrogen phosphate.

10. A solid bioabsorbable material according to any preceding claim, wherein said material provides an equilibrium pH in biological saline solution at 25°C of from 4.5 to 6.5.

11. A solid bioabsorbable material according to any preceding claim, wherein the water-soluble acid and the conjugate base of said buffer system are both solids when anhydrous at 25°C.

12. A solid bioabsorbable material according to any preceding claim, wherein the protein matrix comprises collagen.

13. A solid bioabsorbable material according to claim 12, wherein the protein matrix consists essentially of collagen.

14. A solid bioabsorbable material according to any preceding claim in which the protein matrix is in the form of a film or a web.

15. A solid bioabsorbable material according to any of claims 1 to 13 in which the protein matrix is in the form of a freeze-dried or solvent-dried sponge.

16. A wound dressing or implant comprising a solid bioabsorbable material according to any of claims 1 to 15.

17. Use of a solid bioabsorbable material according to any of claims 1 to 15 for the preparation of a dressing or implant for the treatment of a wound.

18. Use according to claim 17, wherein the wound is a chronic wound.
